# EUROPEAN PATENT SPECIFICATION

(11) **EP 2 358 355 B1**
(45) Date of publication and mention of the grant of the patent: **06.11.2013**
(21) Application number: 09760395.5
(22) Date of filing: 18.11.2009
(51) Int. Cl.: A61K 9/08, A61K 9/10, A61K 47/10, A61K 47/14, A61K 31/44

(54) **PHARMACEUTICAL COMPOSITION OF A POTENT HCV INHIBITOR FOR ORAL ADMINISTRATION**
PHARMAZEUTISCHE ZUSAMMENSETZUNG EINES POTENTEN HCV-HEMMERS ZUR ORALEN VERABREICHUNG
COMPOSITION PHARMACEUTIQUE D'UN INHIBITEUR PUISSANT DU VHC POUR UNE ADMINISTRATION ORALE

(30) Priority: 21.11.2008 US 116789 P
(43) Date of publication of application: 24.08.2011
(73) Proprietor: Boehringer Ingelheim International GmbH, 55216 Ingelheim (DE)
(72) Inventor: CHEN, Feng-Jing, Ridgefield, Connecticut 06877 (US); GEL, Juan Francisco, Buenos Aires C1429BMS (AR); VILLAGRA, Maria Fernanda, Buenos Aires C1429BMS (AR)
(74) Representative: Zwickl, Markus
(86) International application number: PCT/US2009/064908
(87) International publication number: WO 2010/059667

(56) References cited:
- WO-A1-2004/103996
- US-A1- 2005 267 151
- US-B1- 6 268 207

## Description

### BACKGROUND OF THE INVENTION

### 1. TECHNICAL FIELD

The invention relates to a pharmaceutical composition of a potent hepatitis C viral (HCV) inhibitor for oral administration.

### 2. BACKGROUND INFORMATION

The following Compound (1): 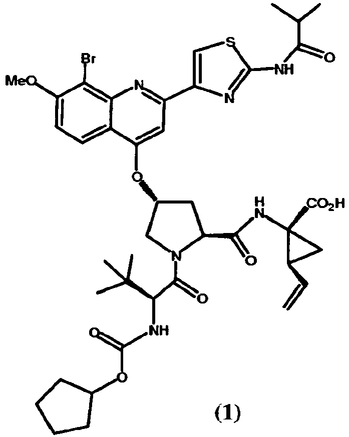 is known as a selective and potent inhibitor of the HCV NS3 serine protease. Compound (1) is a zwitterionic compound and falls within the scope of the acyclic peptide series of HCV inhibitors disclosed in U.S. Patents 6,323,180, 7,514,557 and 7,585,845. Compound (1) is disclosed specifically as Compound # 1055 in U.S. Patent 7,585,845, and as Compound # 1008 in U.S. Patent 7,514,557. Compound (1) can be prepared according to the general procedures found in the above-cited references, which are herein incorporated by reference. Preferred forms of Compound (1) include the crystalline forms, in particular the crystalline sodium salt form, which can be prepared as described in the examples section herein.

Compound (1) may also be known by the following alternate depiction of its chemical structure, which is equivalent to the above-described structure: 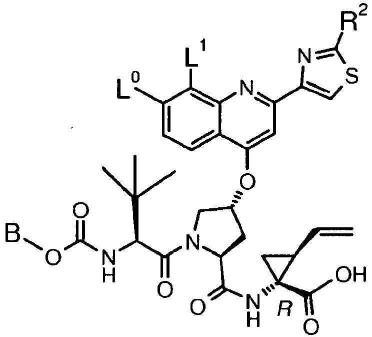 wherein **B** is 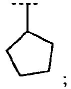 **L⁰** is MeO-; **L¹** is Br; and **R²** is

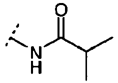

U.S. Patent 6,531,139 and the corresponding published International Application WO9906024 describe a pharmaceutical composition which comprises a lipophilic, pharmaceutically active agent, a lipid which is a mixture of mono- and diglycerides, a solvent and a surfactant. A number of pharmaceutically acceptable solvents are listed, including polyethylene glycol, although propylene glycol is stated to be the preferred solvent. A number of pharmaceutically acceptable surfactants are listed, with Cremophor RH40® or Cremophor EL® being preferred. Vitamin E TPGS is not included in the listing of pharmaceutically acceptable surfactants. These cited references indicate that the composition described therein, which is a liquid, may be used to fill capsules for oral administration, and that it may also be in the form of a liquid solution for oral, parenteral, rectal or topical application.

The disclosures of U.S. Patent 6,121,313 and the corresponding published International Application WO9906043 are essentially the same as that of U.S. Patent 6,531,139 and the corresponding published International Application WO9906024 described above, but the pharmaceutically active agent is limited to certain pyranones.

U.S. Patent 6,231,887 and the corresponding published International Application WO9906044 describe a pharmaceutical composition which comprises a pyranone as a pharmaceutically active agent, a basic amine, a solvent and a surfactant, and optionally a lipid which is a mixture of mono- and diglycerides. A number of pharmaceutically acceptable solvents are listed, including polyethylene glycol, although propylene glycol is stated to be the preferred solvent. A number of pharmaceutically acceptable surfactants are listed, with Cremophor RH40® or Cremophor EL® being preferred. Vitamin E TPGS is not included in the listing of pharmaceutically acceptable surfactants. It is indicated that the composition thus described, which is a liquid, may be used to fill capsules for oral administration, and that it may also be in the form of a liquid solution for oral, parenteral, rectal or topical application.

U.S. Patent 6,555,558 and the corresponding published International Application WO0236110 describe a pharmaceutical composition which comprises a pyranone protease inhibitor (specifically including but not limited to tipranavir), a surfactant, a polyethylene glycol solvent, a lipid which is a mixture of mono- and diglycerides and, optionally, a basic amine. The composition is substantially free of ethanol and propylene glycol. A number of pharmaceutically acceptable surfactants are listed, with Cremophor EL® being preferred. Vitamin E TPGS is not included in the listing of pharmaceutically acceptable surfactants. It is indicated that the composition thus described, which is a liquid, is particularly suitable for filling soft gelatin capsules intended for oral administration.

Vitamin E-TPGS (d-Alpha Tocopheryl Polyethylene Glycol 1000 Succinate) is a water soluble form of vitamin E and has been recognized as an excipient to promote emulsification of lipophilic substances, acting as a non-ionic surfactant, and in improving the bioavailability of certain drugs.

For example, in The Lancet, 1991. 338, 212-214 Sokol R. J. et al teaches that coadministration of Vitamin E-TPGS with cyclosporin improves the bioavailability of cyclosporin.

U.S. Patent 6193985 and the corresponding published International Application WO9531217 describe the use of tocopherols as solvents and/or surfactants of drugs that are substantially insoluble in water, in particular for the preparation of topical formulations. Use of Vitamin E-TPGS is specifically mentioned at pages 7-8 and 12 as an surfactant for use in formulations containing high levels of alpha.-tocopherol as the lipid layer. Examples of formulations for topical administration disclosed containing Vitamin E-TPGS, such as Examples 1 to 5, typically comprises a lipid layer (an .alpha.-tocopherol), the drug and Vitamin E-TPGS, in quantities of less than 25% w/w of the formulation, as an surfactant.

WO96/36316 teaches that Vitamin E-TPGS can be used for the enhanced delivery of lipophilic compounds as a self-emulsifying preconcentrate formulation comprising a) a lipophilic drug (a cyclosporin is specifically exemplified), b) vitamin E-TPGS and c) a lipophilic phase. Typical examples of formulations disclosed, such as Examples 2 and 4, contain less than 14% w/w Vitamin E-TPGS as an surfactant, a lipid layer and the drug. There is no reference to formulation of HIV protease inhibitors.

Finally, U.S. Patent 6,730,679, the corresponding published International Application WO9735587 and Yu et al., Pharm Res. 1999 Dec;16(12):1812-7 describe pharmaceutical compositions containing amprenavir, an HIV protease inhibitor, and Vitamin E-TPGS.

It is believed that there are not yet any formulations of Compound (1) which are particularly well adapted for oral administration in the form of an unencapsulated liquid.

Such a formulation would be particularly suitable for pediatric patients and also for adults who have difficulty swallowing solids.

Thus, it is the object of the present invention to provide such a liquid formulation of Compound (1).

### BRIEF SUMMARY OF THE INVENTION

The present invention provides a pharmaceutically acceptable oral formulation of Compound (1), or a pharmaceutically acceptable salt thereof, in the form of a solution for oral administration, according to claims 1 and 9.

Based upon the physicochemical characteristics of the drug substance, the scope for the development was to formulate a solution with the ability to form an emulsion, microemulsion or micellar solution upon contact with aqueous media. The formulation comprises at least one solvent to enhance solubility of the drug and at least one surfactant with a hydrophilic/lipophilic balance (HLB) > 10 added to maintain the drug substance in a dissolved state upon dilution in simulated GI fluids. The formulation of the invention can further contain water as a co-solvent and taste masking components, such as sweeteners and flavors. An antioxidant may be added to prevent drug substance oxidation. Two examples of composition of this formulation, in two different strengths, are shown in Table I.

**Table I. Compound (1) NA Oral Solution Formulation (F330 and 335)**

| Ingredient | F 330 % w/w | F 335 %w/w | Function |
|---|---|---|---|
| Compound (1) sodium salt | 2.2 | 4.4 | Drug Substance |
| Polyethylene Glycol 400 | 36.5 | 34.3 | Solvent |
| Propylene Glycol | 5.4 | 5.4 | Solvent |
| Vitamin E Polyethylene Glycol Succinate | 29.6 | 29.6 | Surfactant |
| Water, Purified | 22.4 | 22.4 | Solvent |
| Sucralose | 1.9 | 1.9 | Sweetening agent |
| Butter toffee | 2.0 | 2.0 | Flavor |
| Total Weight | 100.0 | 100.0 | |

Sometimes due to physicochemical characteristics, the drug substance in combination with the surfactant may solidify or may not be fluid enough for oral administration. One of the objects of the present invention was to obtain a solution well adapted for oral administration in an unencapsulate form. It is preferred that such formulation would be flowable at room temperature, thereby rendering it suitable for oral administration in a liquid form and to facilitate dosing. It has been found that the addition of at least one cosolvent in combination with the surfactant helps to maintain the present formulation as a liquid flowable solution at room temperature, thereby achieving the advantages mentioned above.

The drug substance compound is present in the composition at a concentration level such as to provide flexibility of dosing by allowing one to change the dose administered by changing the volume. This flexibility with respect to dosing provides a wide range of dosing from a low dose for administration to small children to a high dose for adults who are unable to swallow a solid dosage form.

### DETAILED DESCRIPTION OF THE INVENTION

The liquid composition of the present invention comprises:
(a) Compound (1), or a pharmaceutically acceptable salt thereof: 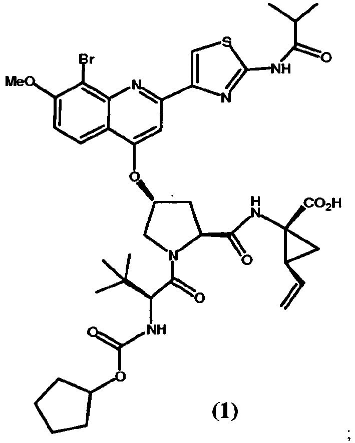
(b) at least one surfactant; and
(c) at least one pharmaceutically acceptable solvent;
and wherein the composition is substantially free of lipid.

Preferred forms of Compound (1) that may be used in the formulation include its crystalline forms, in particular the crystalline sodium salt form of Compound (1).

Surfactants suitable for use in the composition of the present invention include surfactants having a hydrophilic/lipophilic balance (HLB) of greater than 10. Examples of suitable surfactants include Vitamin E TPGS, a polyethoxylated castor oil (e.g. CREMOPHOR^{®} EL), a polyoxyl hydrogenated castor oil (e.g. CREMOPHOR^{®} RH), a polyoxyethylene sorbitan fatty ester (e.g. polysorbate 80), a caprylocaproyl macrogolglyceride (e.g. LABRASOL^{®}) or a mixture thereof. A preferred surfactant is Vitamin E TPGS. The surfactant comprises 10% to 30% by weight of the total composition.

Different ranges of surfactant and drug substance (DS) should result in different aqueous dispersions. Examples of these compositions are showed in Table II below:

**Table II. Compound (1) NA Oral Solution Formulations with different Vitamin E TPGS to DS ratio**

| Ingredient | F 296 % w/w | F 145 % w/w | F 331 % w/w | F363 % w/w | F 355 % w/w | F 332 % w/w | F 333 % w/w | F 334 % w/w |
|---|---|---|---|---|---|---|---|---|
| Vitamin E TPGS : DS ratio | 8.1 | 4.3 | 4.1 | 2.9 | 2.7 | 2.0 | 1.4 | 1 |
| Compound (1) sodium salt | 2.2 | 6.3 | 4.4 | 4.6 | 4.4 | 4.4 | 4.4 | 4.4 |
| Polyethylene Glycol 400 | 56.1 | 42.7 | 45.9 | 50.2 | 50.4 | 54.9 | 57.6 | 59.4 |
| Propylene Glycol | 7.1 | 7.2 | 5.4 | 5.4 | 5.4 | 5.4 | 5.4 | 5.4 |
| Vitamin E Polyethylene Glycol Succinate | 17.7 | 26.8 | 18.0 | 13.5 | 13.5 | 9.0 | 6.3 | 4.5 |
| Water, Purified | 13.3 | 13.4 | 22.4 | 22.4 | 22.4 | 22.4 | 22.4 | 22.4 |
| Sucralose | 1.8 | 1.8 | 1.9 | 1.9 | 1.9 | 1.9 | 1.9 | 1.9 |
| Butter mint | 0.9 | 0.9 | - | | - | - | - | - |
| Butter toffee | 0.9 | 0.9 | 2.0 | 2 | 2.0 | 2.0 | 2.0 | 2.0 |
| Total Weight | 100.0 | 100.0 | 100.0 | 100.0 | 100 | 100 | 100.0 | 100.0 |
| Visual clarity observation upon dispersion 25x in Gastric fluid (pH 1.2) | Clear | Clear | Clear | Clear | Clear | Slightly turbid | Slightly turbid | Turbid |

**Table III. Compound (1) NA Oral Solution Formulations with different Vitamin E TPGS to DS ratio and at a High Drug Load**

| Ingredient | F 145 % w/w | F 180 % w/w | F 181 % w/w |
|---|---|---|---|
| Vitamin E TPGS : DS ratio | 4.3 | 2.9 | 1.4 |
| Compound (1) sodium salt | 6.3 | 6.3 | 6.3 |
| Polyethylene Glycol 400 | 42.7 | 51.7 | 60.6 |
| Propylene Glycol | 7.2 | 7.1 | 7.2 |
| Vitamin E Polyethylene Glycol Succinate | 26.8 | 17.9 | 8.9 |
| Water, Purified | 13.4 | 13.4 | 13.4 |
| Sucralose | 1.8 | 1.8 | 1.8 |
| Butter mint | 0.9 | 0.9 | 0.9 |
| Butter toffee | 0.9 | 0.9 | 0.9 |
| Total Weight | 100.0 | 100.0 | 100.0 |
| Visual clarity observation upon dispersion 25x in Gastric fluid (pH 1.2) | Clear | Slightly turbid | Slightly turbid |

As can be seen from the results in the above Table II, on visual observation the compositions comprising Vitamin E TPGS (surfactant) to Compound (1) sodium salt (drug substance) ratios greater than or equal to 2.7 produced clear dispersion upon dilution in simulated gastric fluid, compositions comprising ratios from 1.4 to 2 produced slightly turbid or translucent dispersions and compositions comprising ratios equal to or lower than 1 produce a suspension having a turbid or "milky" appearance upon dilution in simulated gastric fluid. As can be seen from the results in Table III, however, at a higher drug substance loading a higher surfactant to drug substance ratio is necessary to provide a clear dispersion. Thus, at a high drug loading of 6.3 % compositions comprising surfactant to drug substance ratios from 1.4 to 2.9 produced slightly turbid or translucent dispersions and only at the higher ratio of 4.3 was a clear dispersion obtained. Accordingly, embodiments of the present invention include:
(b) compositions wherein the weight ratio of surfactant to drug substance is greater than or equal to 2.7; and
(c) compositions wherein the weight ratio of surfactant to drug substance is greater than or equal to 4.3

Said embodiments include: compositions containing drug substance in an amount less than or equal to 4.6% and the weight ratio of surfactant to drug substance is greater than or equal to 2.7; and compositions containing drug substance in an amount less than or equal 6.3% and the weight ratio of surfactant to drug substance is greater than or equal to 4.3.

Additional preferred embodiments include any of the above embodiments wherein the surfactant is Vitamin E TPGS.

Compositions of the present invention form a clear dispersion upon dilution in simulated gastric fluid. When the formulation forms a clear, translucent or only slightly turbid dispersion on dilution, this is indicative that there has been no or only a limited amount of Compound (1) precipitation and that the active ingredient has remained substantially solubilized. Such systems are preferable in that one would generally expect them to result in a higher bioavalibility of the active ingredient upon ingestion as compared to a turbid dispersion where the active ingredient has substantially precipitated.

The clarity of the final dispersion can be verified by well known methods in the art. The clarity can be determined by measuring the size of droplets and particles using laser light scattering methods (e.g. dynamic light scattering or static light scattering) which are well known in the art. Different ratios of surfactant to drug substance will produce different particle/droplet sizes and different levels of clarity. The smaller the droplet size of the emulsion, microemulsion or micellar particles, the more clear will be the solution formed. A typical value of mean particle size for a clear final dispersion can be less than 1 µm, while for a slightly turbid or turbid dispersion the value of particle sizes will be higher than 1 µm. Examples of composition having different clarity and droplet or particle sizes are shown in example 7.

Thus, the composition has a mean particle size of less than 1 µm upon dilution in simulated gastric fluid.

Pharmaceutically acceptable solvents suitable for use in the context of the present invention are propylene glycol, polypropylene glycol, polyethylene glycol (such as low molecular weight polyethylene glycol including but not limited to PEG300, 400, 600, etc.), glycerol, ethanol, triacetin, dimethyl isosorbide, glycofurol, propylene carbonate, water, dimethyl acetamide or a mixture thereof. In one embodiment, at least one solvent is a low molecular weight polyethylene glycol, for example, polyethylene glycol 300, polyethylene glycol 400, polyethylene glycol 600, or mixtures thereof. The preferred solvent is a mixture of water, polyethylene glycol having a mean molecular weight of greater than 300 but lower than 600 and propylene glycol. Still more preferred as solvent is a mixture of water, propylene glycol and polyethylene glycol 400. In another preferred embodiment, the solvent is a mixture of water and polyethylene glycol 400. The solvent, or mixture of solvents, comprises 10% to 90% by weight of the total composition, with preferred amounts of 60% to 90% by weight of the total composition.

In a preferred embodiment, the water co-solvent is present in the composition in an amount of 0 to 50% by weight of the total composition, more preferably from 0 to 30% by weight of the total composition, even more preferably from 5 to 20% by weight of the total composition.

The compositions of the present invention are preferably substantially free of propylene glycol. In this context, "substantially free" means less than or equal to 8% by weight, more preferably less than or equal to 2% by weight, of propylene glycol in the composition. In a preferred embodiment the composition of the present invention does not contain any propylene glycol.

The compositions of the present invention are also preferably substantially free of amines. In this context, "substantially free" means less than or equal to 2% by weight, more preferably less than or equal to 1 % by weight, even more preferably less than or equal to 0.5% by weight, of amine in the composition. In a preferred embodiment the composition of the present invention does not contain any amine.

The compositions in accordance with the invention are substantially free of lipid in the composition, because these compounds could have a significant influence in the taste. So by avoiding the addition or significant reduction of such substances, an appropriate palatability can be achieved, particularly for pediatric use. In this context, "substantially free" means less than or equal to 5% by weight, more preferably less than or equal to 2% by weight, of lipid in the composition. In a preferred embodiment the composition of the present invention does not contain any lipid.

The composition in accordance with the invention optionally includes further excipients, such as antioxidants (e.g. α-tocopherol, propyl gallate, ascorbic palmitate, BHT, BHA or mixtures thereof) and/or sweetening (e.g. sucralose, accesulfame potassium, sodium saccharin, or mixtures thereof) and flavoring agents (e.g. butter toffee, buttermint, bubble gum, grape, cherry, strawberry or mixtures thereof). Thus, for example, it is preferred to include agents to sweeten or flavor the formulation. Those of ordinary skill in the pharmaceutical art will know how to select acceptable sweetening or flavoring agents.

In one embodiment, the pharmaceutical composition comprises:
(a) 2% to 10% by weight of Compound (1), or a pharmaceutically acceptable salt thereof;
(b) 10% to 30% by weight of surfactant; and
(c) 60% to 90% by weight of solvent or mixture of solvents; and
wherein the composition is substantially free of lipid, or more preferably does not contain any lipid.

In another embodiment, the pharmaceutical composition comprises:
(a) 2% to 10% by weight of Compound (1), or a pharmaceutically acceptable salt thereof;
(b) 10% to 30% by weight of Vitamin E TPGS; and
(c) 60% to 90% by weight of a mixture of water, propylene glycol and polyethylene glycol 400 ; and
wherein the composition is substantially free of lipid, or more preferably does not contain any lipid.

In another embodiment, the pharmaceutical composition comprises:
(a) 2% to 10% by weight of Compound (1), or a pharmaceutically acceptable salt thereof;
(b) 10% to 30% by weight of Vitamin E TPGS; and
(c) 60% to 90% by weight of a mixture of water and polyethylene glycol 400; and
wherein the composition is substantially free of lipid, or more preferably does not contain any lipid.

Additional embodiments include embodiments, wherein the composition is (1) substantially free of propylene glycol or does not contain propylene glycol, and/or (2) substantially free of an amine or does not contain an amine.

An example of a methodology for manufacture the inventions is as follows: mix solvents at a temperature or 40°C - 50°C, add surfactant and mix. Then add the drug substance and mix until complete dissolution. Add the sweetener dissolved in water and mix. Lower the temperature up to 35-37°C, add the flavors and mix.

The self-dispersing formulations in accordance with the present invention generate micellar solutions when mixed with aqueous media. The formulation can be mixed with an aqueous medium such as water, fruit juice or the like, prior to ingestion. The formulation can be ingested in liquid form so that it will mix with gastric fluid, forming a micelar solutions in situ. In certain circumstance, the Compound (1) may precipitate out of solution when the formulation is mixed with gastric fluid, resulting in the formation of a suspension having a turbid or "milky" appearance.

The compositions in accordance with the present invention are useful in the treatment of Hepatitis C viral (HCV) infection and can be administered in accordance with the general protocols as described in U.S. Patent 7,585,845. The skilled physician can select appropriate dosing for any particular patient by following the general dosing guidelines found in said patent publication and using sound medical judgment, taking into consideration the age, size, general health, severity of the condition and other characteristics of the particular patient to be treated.

Examples 1-5 describe the preparation of various crystalline forms of Compound (1)

### Example I - Preparation of Type A crystalline form of Compound (1)

Amorphous Compound (**1**) (Batch 7, 13.80g) was added to a 1000 ml three neck flask. Absolute ethanol (248.9g) was added to the flask. While stirring, the contents of the flask were heated at 60 degrees C/hr to ∼ 74 degrees C. (Solids do not dissolve at 74 degrees C). Water (257.4g) was then added linearly over 4 hr to the resulting slurry while stirring and maintaining the temperature at 74 degrees C. After the water addition was complete, the temperature was reduced linearly to ambient temperature at 8 degrees C/hr and then held at ambient temperature for 6 hrs while stirring. The resulting solids were collected by filtration and washed with 50 ml of 1/1 (w/w) EtOH/Water. The wet solids were dried on the funnel for 30 minutes by drawing N₂ through the cake. (XRPD analysis on this sample indicates that the pattern is similar to the EtOH solvate). The solids were then dried at 65-70 degrees C under vacuum (P = 25 in Hg) and a nitrogen bleed for 1.5 hr. The resulting solids (12.6g, 95.5 % corrected yield) were conformed by XRPD as being Type A Compound (**1**).

### Example 2 - Preparation of the Sodium Salt of Compound (1) - Method 1

2.1 g of amorphous sodium salt of Compound (1) and 8.90g of acetone was added to a vial and stirred at ambient temperature for 3 hr. The slurry was filtered off mother liquors and the resulting solids were dried for 20 minutes under nitrogen flow for 20 minutes. 1.51 g of crystalline sodium salt of Compound (1) as solids was collected.

### Example 3 - Preparation of the Sodium Salt of Compound (1) - Method 2

15.6 g of Type A of Compound (1), 175 ml of acetone and 3.6 ml of water was added to a 250 ml reactor and heated to 53 degrees C to dissolve the solids. 900 ul of 10.0 N NaOH was added to reactor and the solution was seeded with Type A. The seeded solution was stirred at 53 degrees C for 10 minutes. A second 900 ul portion of 10.0 N NaOH was added and the system was stirred at 53 degrees C for 30 minutes over which a slurry developed. The slurry was cooled to 19 degrees C at a cooling rate of 15 degrees C per hour and held overnight at 19 degrees C. The final resulting slurry was filtered and the wet solids were washed with 15 ml of acetone. Dried solids for 1 hr at 52 degrees C under vacuum with a nitrogen flow and then exposed the solids to lab air for one hour. Collected 12.1 g of Compound (1) crystalline sodium salt solids.

### Example 4 - Preparation of the Sodium Salt of Compound (1) - Method 3

25.4 Kg of amorphous Compound (1), 228 L of THF and 11.1 Kg of 10 wt % NaOH (aq) was added to a reactor. The components were mixed at 25 degrees C to dissolve all solids. The resulting solution was filtered and the reactor and filter was washed with 23 L of THF. 180 L of solvent was removed using atmospheric distillation at 65 degrees C. 195 L of MIBK was added and 166 L of solvent was removed by vacuum distillation at ∼ 44 degrees C. 161 L of MIBK and 0.41 Kg of water was added back to the reactor and the contents were heated to 70 degrees C. 255 g of Compound (1) sodium salt seeds were added at 70 degrees C and 1.42 L of water was added over 1.5 hours. After the water addition the slurry was held at 70 degrees C for 45 minutes and then cooled to 45 degrees C over 1 hr. The resulting slurried was filtered and washed with 64 L of MIBK containing ∼0.8 weight to water. The wet cake was dried at 55 degrees C to give ∼ 25 Kg of crystalline sodium salt of Compound (1).

### Example 5 - Preparation of the Sodium Salt of Compound (1) - Method 4

2.00 g of amorphous Compound (1), 9.96g of THF and 0.11 g of water was added to a reactor and stirred at ambient temperature to dissolve solids. 0.820ml of 21 weight% NaOET in ethanol was added drop-wise while stirring the solution to get solution A. 15.9 g of n-BuAc and 160 ul of water was added to a second reactor and heated to 65 degrees C (solution B). 2.56 g of Solution A was added to Solution B at 65 degrees C and the resulting mixture was seeded with 40 mg of Compound (1) sodium salt seeds. The seeded mixture was aged at 65 degrees C for 45 minutes. 2.56 g of Solution B was added to Solution A and aged for 45 minutes in four separate intervals. After the final addition and aging, the slurry was cooled to 50 degrees C over 1 hour and filtered. The wet cake was washed with 6 ml of n-BuAc containing 0.5 weight % water. The final solids were dried at 50 degrees C under vacuum using a nitrogen purge. Compound (1) crystalline sodium salt solids were collected.

The following example provides additional examples of pharmaceutical formulations of the present invention.

### Example 6: Pharmaceutical Compositions of Compound (1) sodium salt.

The following ingredients in Table IV, V, VI, VII, VIII and IX were mixed to form a liquid formulation.

**Table IV: compositions of Compound (1) sodium salt oral solution comprising different drug loading**

| Ingredient | F 325 % w/w | F 324 % w/w | F 145 % w/w |
|---|---|---|---|
| Compound (1) sodium salt | 2.2 | 4.4 | 6.3 |
| Polyethylene Glycol 400 | 36.5 | 34.3 | 42.7 |
| Propylene Glycol | 5.4 | 5.4 | 7.2 |
| Vitamin E Polyethylene Glycol Succinate | 29.6 | 29.6 | 26.8 |
| Water, Purified | 22.4 | 22.4 | 13.4 |
| Sucralose | 1.9 | 1.9 | 1.8 |
| Butter toffee | 2.0 | 2.0 | 0.9 |
| Buttermint | - | - | 0.9 |
| Total Weight | 100.0 | 100.0 | 100.0 |

**Table V: compositions of Compound (1) sodium salt oral solution comprising different ratios of Vitamin E TPGS to DS**

| Ingredient | F 296 % w/w | F 145 % w/w | F 331 % w/w | F 355 % w/w | F 332 % w/w | F 333 % w/w | F 334 % w/w |
|---|---|---|---|---|---|---|---|
| Compound (1) sodium salt | 2.2 | 6.3 | 4.4 | 4.4 | 4.4 | 4.4 | 4.4 |
| Polyethylene Glycol 400 | 56.1 | 42.7 | 45.9 | 50.4 | 54.9 | 57.6 | 59.4 |
| Propylene Glycol | 7.1 | 7.2 | 5.4 | 5.4 | 5.4 | 5.4 | 5.4 |
| Vitamin E Polyethylene Glycol Succinate | 17.7 | 26.8 | 18.0 | 13.5 | 9.0 | 6.3 | 4.5 |
| Water, Purified | 13.3 | 13.4 | 22.4 | 22.4 | 22.4 | 22.4 | 22.4 |
| Sucralose | 1.8 | 1.8 | 1.9 | 1.9 | 1.9 | 1.9 | 1.9 |
| Butter mint | 0.9 | 0.9 | - | - | - | - | - |
| Butter toffee | 0.9 | 0.9 | 2.0 | 2.0 | 2.0 | 2.0 | 2.0 |
| Total Weight | 100.0 | 100.0 | 100.0 | 100 | 100 | 100.0 | 100.0 |
| Dispersion 25x in Gastric fluid (pH 1.2) | Clear | Clear | Clear | Clear | Slightly turbid | Slightly turbid | Turbid |

**Table VI: compositions of Compound (1) sodium salt oral solution comprising different combination of Solvents**

| Ingredient | F 305 % w/w | F 304 % w/w | F 327 g/% w/w | F 336 g% w/w | F 299 g% w/w | F 326 % w/w | F 213 % w/w | F 212 % w/w |
|---|---|---|---|---|---|---|---|---|
| Compound (1) sodium salt | 2.2 | 2.2 | 2.2 | 4.4 | 4.4 | 4.4 | 6.3 | 6.3 |
| Polyethylene Glycol 400 | 45.1 | 42.4 | 40.8 | 39.7 | 43.0 | 38.5 | 23.1 | 32.0 |
| Propylene Glycol | - | 6.0 | 7.1 | - | 5.7 | 7.1 | 26.8 | 17.9 |
| Vitamin E Polyethylene Glycol Succinate | 32.2 | 30.2 | 28.3 | 29.6 | 28.6 | 28.2 | 26.8 | 26.8 |
| Water, Purified | 16.1 | 15.1 | 18.0 | 22.4 | 14.4 | 17.9 | 13.4 | 13.4 |
| Sucralose | 2.2 | 2.1 | 1.8 | 1.9 | 1.9 | 1.9 | 1.8 | 1.8 |
| Butter mint | 1.1 | 1.0 | 0.9 | - | 1.0 | 1.0 | 0.9 | 0.9 |
| Butter toffee | 1.1 | 1.0 | 0.9 | 2.0 | 1.0 | 1.0 | 0.9 | 0.9 |
| Total Weight | 100.0 | 100.0 | 100.0 | 100.0 | 100.0 | 100.0 | 100.0 | 100.0 |

**Table VII: compositions of Compound (1) sodium salt oral solution comprising low levels of Water**

| Ingredient | F 343 % w/w | F 344 % w/w | F 345 % w/w |
|---|---|---|---|
| Compound (1) sodium salt | 4.4 | 4.4 | 4.4 |
| Polyethylene Glycol 400 | 76.3 | 71.3 | 66.3 |
| Propylene Glycol | 5.4 | 5.4 | 5.4 |
| Vitamin E Polyethylene Glycol Succinate | 5 | 5 | 10 |
| Water, Purified | 5 | 10 | 10 |
| Sucralose | 1.9 | 1.9 | 1.9 |
| Butter toffee | 2.0 | 2.0 | 2.0 |
| Total Weight | 100.0 | 100.0 | 100.0 |

**Table VIII: compositions of Compound (1) sodium salt oral solution comprising different Surfactants**

| Ingredient | F 170 % w/w | F 172 % w/w | F 340 % w/w |
|---|---|---|---|
| Compound (1) sodium salt | 6 | 6 | 6.3 |
| Polyethylene Glycol 400 | 49.6 | 49.6 | 42.8 |
| Propylene Glycol | 6.8 | 6.8 | 7.1 |
| Vitamin E Polyethylene Glycol Succinate | 12.8 | 12.8 | - |
| Cremophor EL | 8.5 | - | - |
| Cremophor RH 40 | - | 8.5 | 26.8 |
| Water, Purified | 12.8 | 12.8 | 13.4 |
| Sucralose | 1.7 | 1.7 | 1.8 |
| Butter mint | 0.9 | 0.9 | 0.9 |
| Butter toffee | 0.9 | 0.9 | 0.9 |
| Total Weight | 100.0 | 100.0 | 100.0 |

**Table IX: compositions of Compound (1) sodium salt oral solution comprising amines**

| Ingredient | F 383 % w/w | F 382 % w/w |
|---|---|---|
| Compound (1) sodium salt | 4.60 | 4.60 |
| Polyethylene Glycol 400 | 54.6 | 54.3 |
| Propylene Glycol | 5.4 | 5.4 |
| Vitamin E Polyethylene Glycol Succinate | 13.5 | 13.5 |
| Water, Purified | 17.9 | 17.9 |
| Tris | 0.2 | 0.4 |
| Sucralose | 1.9 | 1.9 |
| Butter toffee | 2.0 | 2.0 |
| Total Weight | 100.0 | 100.0 |

### Example 7: Pharmaceutical Compositions of Compound (1) sodium salt.

The following ingredients in Table X were mixed to form a liquid formulation. A sample of 10 mL of such compositions were dispersed and agitated with 250 mL of Gastric fluid (pH1.2) for 1 hour. A sample of the resulting dispersion was measured either by static light scattering or alternatively by dynamic light scattering (known also as photon correlation spectroscopy or PCS). Visual observation and particle size results are shown in Table X.

**Table X: Compositions of Compound (1) sodium salt oral solution comprising different ratios of Vitamin E TPGS to DS**

| Ingredient | F 331 % w/w | F 332 % w/w | F 333 % w/w |
|---|---|---|---|
| Compound (1) sodium salt | 4.4 | 4.4 | 4.4 |
| Polyethylene Glycol 400 | 45.9 | 54.9 | 57.6 |
| Propylene Glycol | 5.4 | 5.4 | 5.4 |
| Vitamin E Polyethylene Glycol Succinate | 18.0 | 9.0 | 6.3 |
| Water, Purified | 22.4 | 22.4 | 22.4 |
| Sucralose | 1.9 | 1.9 | 1.9 |
| Butter mint | - | - | - |
| Butter toffee | 2.0 | 2.0 | 2.0 |
| Total Weight | 100.0 | 100 | 100.0 |
| Dispersion 25x in Gastric fluid (pH 1.2) | Clear | Slightly turbid | Slightly turbid |
| Mean particle size measured by dynamic light scattering or PCS | 0.064 µm | - | - |
| Mean particle size (measured by static light scattering) | - | 19.21 µm | 16.41 µm |

## Claims

1. A liquid pharmaceutical composition which comprises:
(a) less than or equal to 4.6% by weight of Compound (1), or a pharmaceutically acceptable salt thereof: 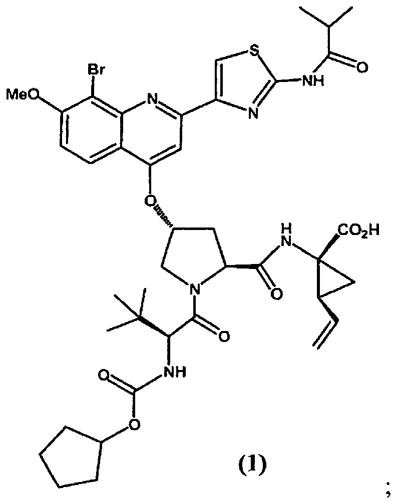
(b) at least one 10% to 30% by weight of surfactant; and
(c) at least one 60% to 90% by weight of a pharmaceutically acceptable solvent; wherein the weight ratio of surfactant to Compound (1) or pharmaceutically acceptable salt thereof is greater than or equal to 2.7;
and wherein the composition is substantially free of lipid,
and wherein the composition forms a clear dispersion having a mean particle size of less than 1 µm upon dilution in simulated gastric fluid.

2. A pharmaceutical composition in accordance with claim 1, wherein the surfactant has a hydrophilic/lipophilic balance of greater than 10.

3. A pharmaceutical composition in accordance with claim 1, wherein the surfactant is Vitamin E TPGS, a polyethoxylated castor oil, a polyoxyl hydrogenated castor oil, a polyoxyethylene sorbitan fatty ester, a caprylocaproyl macrogolglyceride or a mixture thereof.

4. A pharmaceutical composition in accordance with claim 1, wherein the pharmaceutically acceptable solvent is propylene glycol, polypropylene glycol, polyethylene glycol, glycerol, ethanol, triacetin, dimethyl isosorbide, glycofurol, propylene carbonate, water, dimethyl acetamide or mixtures thereof.

5. A pharmaceutical composition in accordance with claim 1, wherein the solvent is a mixture of water, polyethylene glycol having a mean molecular weight of greater than 300 but lower than 600 and propylene glycol.

6. A pharmaceutical composition in accordance with claim 1, wherein the pharmaceutical composition does not contain any lipid.

7. A pharmaceutical composition in accordance with claim 1, wherein the pharmaceutical composition is substantially free of propylene glycol.

8. A pharmaceutical composition in accordance with claim 1, wherein the pharmaceutical composition is substantially free of an amine.

9. A liquid pharmaceutical composition which comprises:
(a) less than or equal to 6.3% by weight of Compound (1), or a pharmaceutically acceptable salt thereof: 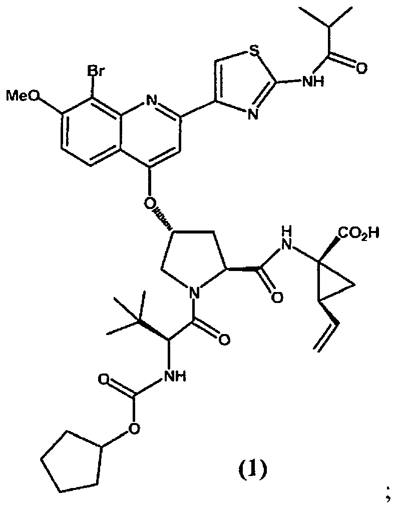
(b) at least one 10% to 30% by weight of surfactant; and
(c) at least one 60% to 90% by weight of a pharmaceutically acceptable solvent;
wherein the weight ratio of surfactant to Compound (1) or pharmaceutically acceptable salt thereof is greater than or equal to 4.3;
and wherein the composition is substantially free of lipid,
and wherein the composition forms a clear dispersion having a mean particle size of less than 1 µm upon dilution in simulated gastric fluid.

10. A pharmaceutical composition in accordance with claim 9, wherein the surfactant has a hydrophilic/lipophilic balance of greater than 10.

11. A pharmaceutical composition in accordance with claim 9, wherein the surfactant is Vitamin E TPGS, a polyethoxylated castor oil, a polyoxyl hydrogenated castor oil, a polyoxyethylene sorbitan fatty ester, a caprylocaproyl macrogolglyceride or a mixture thereof.

12. A pharmaceutical composition in accordance with claim 9, wherein the pharmaceutically acceptable solvent is propylene glycol, polypropylene glycol, polyethylene glycol, glycerol, ethanol, triacetin, dimethyl isosorbide, glycofurol, propylene carbonate, water, dimethyl acetamide or mixtures thereof.

13. A pharmaceutical composition in accordance with claim 9, wherein the solvent is a mixture of water, polyethylene glycol having a mean molecular weight of greater than 300 but lower than 600 and propylene glycol.

14. A pharmaceutical composition in accordance with claim 9, wherein the pharmaceutical composition does not contain any lipid.

15. A pharmaceutical composition in accordance with claim 9, wherein the pharmaceutical composition is substantially free of propylene glycol.

16. A pharmaceutical composition in accordance with claim 9, wherein the pharmaceutical composition is substantially free of an amine.

## Patentansprüche

1. Flüssige pharmazeutische Zusammensetzung, die umfasst:
(a) 4,6 Gew.-% oder weniger der Verbindung (1) oder eines pharmazeutisch akzeptablen Salzes hiervon: 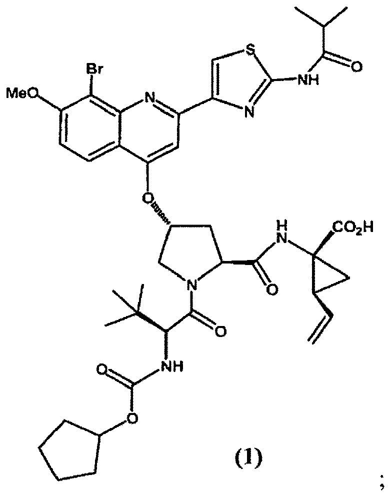
(b) 10 Gew.-% bis 30 Gew.-% mindestens eines oberflächenaktiven Mittels; und
(c) 60 Gew.-% bis 90 Gew.-% mindestens eines pharmazeutisch akzeptablen Lösungsmittels;
wobei das Gewichtsverhältnis des oberflächenaktiven Mittels zu Verbindung (1) oder des pharmazeutisch akzeptablen Salzes hiervon 2,7 oder größer ist;
und wobei die Zusammensetzung im Wesentlichen frei von Lipid ist,
und wobei die Zusammensetzung eine klare Dispersion mit einer mittleren Partikelgröße von weniger als 1 µm nach der Verdünnung bzw. Lösung in simulierter Magenflüssigkeit bildet.

2. Pharmazeutische Zusammensetzung nach Anspruch 1, wobei das oberflächenaktive Mittel einen HLB-Wert (hydrophilic/lipophilic balance) von größer 10 aufweist.

3. Pharmazeutische Zusammensetzung nach Anspruch 1, wobei das oberflächenaktive Mittel Vitamin E TPGS, ein polyethoxyliertes Castoröl, ein polyoxyl-hydriertes Castoröl, einen Polyoxyethylensorbitanfettsäureester, ein Caprylocaproylmakrogolglycerid oder eine Mischung hiervon darstellt.

4. Pharmazeutische Zusammensetzung nach Anspruch 1, wobei das pharmazeutisch akzeptable Lösungsmittel Propylenglykol, Polypropylenglykol, Polyethylenglykol, Glycerin, Ethanol, Triacetin, Dimethylisosorbid, Glycofurol, Propylencarbonat, Wasser, Dimethylacetamid oder Mischungen hiervon darstellt.

5. Pharmazeutische Zusammensetzung nach Anspruch 1, wobei das Lösungsmittel eine Mischung aus Wasser, Polyethylenglykol mit einem mittleren Molekulargewicht größer als 300 aber kleiner als 600 und Propylenglykol darstellt.

6. Pharmazeutische Zusammensetzung nach Anspruch 1, wobei die pharmazeutische Zusammensetzung keinerlei Lipid enthält.

7. Pharmazeutische Zusammensetzung nach Anspruch 1, wobei die pharmazeutische Zusammensetzung im Wesentlichen frei von Propylenglykol ist.

8. Pharmazeutische Zusammensetzung nach Anspruch 1, wobei die pharmazeutische Zusammensetzung im Wesentlichen frei von Amin ist.

9. Flüssige pharmazeutische Zusammensetzung, die umfasst:
(a) 6,3 Gew.-% oder weniger der Verbindung (1) oder eines pharmazeutisch akzeptablen Salzes hiervon: 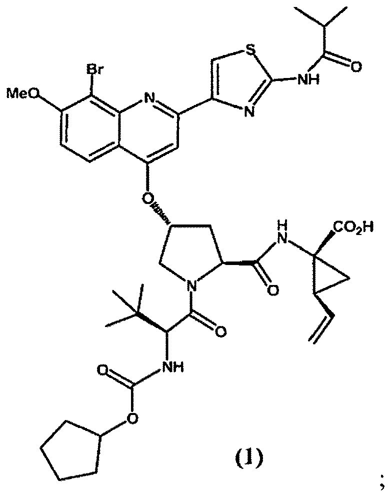
(b) 10 Gew.-% bis 30 Gew.-% mindestens eines oberflächenaktiven Mittels; und
(c) 60 Gew.-% bis 90 Gew.-% mindestens eines pharmazeutisch akzeptablen Lösungsmittels;
wobei das Gewichtsverhältnis des oberflächenaktiven Mittels zu Verbindung (1) oder des pharmazeutisch akzeptablen Salzes hiervon 4,3 oder größer ist;
und wobei die Zusammensetzung im Wesentlichen frei von Lipid ist,
und wobei die Zusammensetzung eine klare Dispersion mit einer mittleren Partikelgröße von weniger als 1 µm nach der Verdünnung bzw. Lösung in simulierter Magenflüssigkeit bildet.

10. Pharmazeutische Zusammensetzung nach Anspruch 9, wobei das oberflächenaktive Mittel einen HLB-Wert (hydrophilic/lipophilic balance) von größer 10 aufweist.

11. Pharmazeutische Zusammensetzung nach Anspruch 9, wobei das oberflächenaktive Mittel Vitamin E TPGS, ein polyethoxyliertes Castoröl, ein polyoxyl-hydriertes Castoröl, einen Polyoxyethylensorbitanfettsäureester, ein Caprylocaproylmakrogolglycerid oder eine Mischung hiervon darstellt.

12. Pharmazeutische Zusammensetzung nach Anspruch 9, wobei das pharmazeutisch akzeptable Lösungsmittel Propylenglykol, Polypropylenglykol, Polyethylenglykol, Glycerin, Ethanol, Triacetin, Dimethylisosorbid, Glycofurol, Propylencarbonat, Wasser, Dimethylacetamid oder Mischungen hiervon darstellt.

13. Pharmazeutische Zusammensetzung nach Anspruch 9, wobei das Lösungsmittel eine Mischung aus Wasser, Polyethylenglykol mit einem mittleren Molekulargewicht größer als 300 aber kleiner als 600 und Propylenglykol darstellt.

14. Pharmazeutische Zusammensetzung nach Anspruch 9, wobei die pharmazeutische Zusammensetzung keinerlei Lipid enthält.

15. Pharmazeutische Zusammensetzung nach Anspruch 9, wobei die pharmazeutische Zusammensetzung im Wesentlichen frei von Propylenglykol ist.

16. Pharmazeutische Zusammensetzung nach Anspruch 9, wobei die pharmazeutische Zusammensetzung im Wesentlichen frei von Amin ist.

## Revendications

1. Composition pharmaceutique liquide qui comprend :
(a) moins de ou 4,6 % en poids de composé (1), ou d'un sel pharmaceutiquement acceptable de celui-ci : 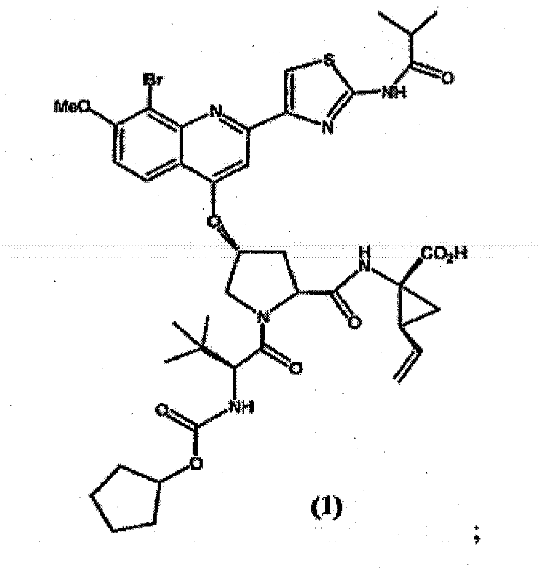
(b) au moins 10 % à 30 % en poids de surfactant ; et
(c) au moins 60 % à 90 % en poids d'un solvant pharmaceutiquement acceptable ;
dans laquelle le rapport pondéral du surfactant sur le composé (1) ou un sel pharmaceutiquement acceptable de celui-ci est supérieur ou égal à 2,7 ;
et dans laquelle la composition est sensiblement dépourvue de lipide,
et dans laquelle la composition forme une dispersion transparente présentant une taille de particule moyenne inférieure à 1 µm après dilution dans un liquide gastrique simulé.

2. Composition pharmaceutique selon la revendication 1, dans laquelle le surfactant présente une balance hydrophile/lipophile supérieure à 10.

3. Composition pharmaceutique selon la revendication 1, dans laquelle le surfactant est la vitamine E-TPGS, une huile de ricin polyéthoxylée, une huile de ricin polyoxylée hydrogénée, un ester gras de polyoxyéthylène sorbitane, un caprylocaproyl-macrogol-glycéride ou un mélange de ceux-ci.

4. Composition pharmaceutique selon la revendication 1, dans laquelle le solvant pharmaceutiquement acceptable est le propylène glycol, le polypropylène glycol, le polyéthylène glycol, le glycérol, l'éthanol, la triacétine, l'isosorbide de diméthyle, le glycofurol, le carbonate de propylène, l'eau, le diméthylacétamide ou des mélanges de ceux-ci.

5. Composition pharmaceutique selon la revendication 1, dans laquelle le solvant est un mélange d'eau, de polyéthylène glycol présentant une masse moléculaire moyenne supérieure à 300 mais inférieure à 600 et de propylène glycol.

6. Composition pharmaceutique selon la revendication 1, dans laquelle la composition pharmaceutique ne contient aucun lipide.

7. Composition pharmaceutique selon la revendication 1, dans laquelle la composition pharmaceutique est sensiblement dépourvue de propylène glycol.

8. Composition pharmaceutique selon la revendication 1, dans laquelle la composition pharmaceutique est sensiblement dépourvue d'amine.

9. Composition pharmaceutique liquide qui comprend :
(a) moins de ou 6,3 % en poids de composé (1), ou d'un sel pharmaceutiquement acceptable de celui-ci : 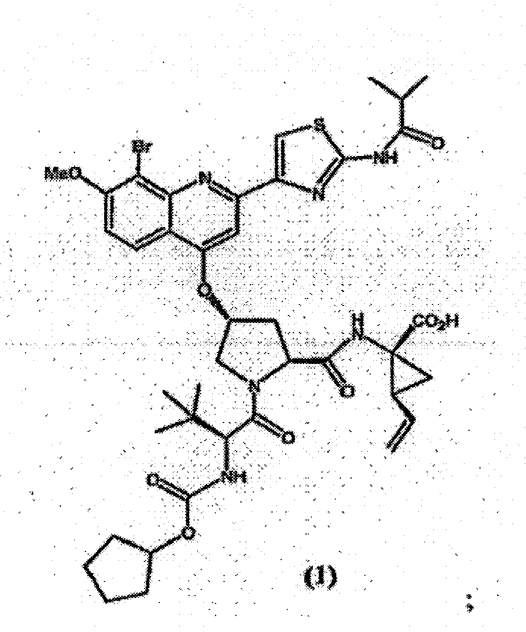
(b) au moins 10 % à 30 % en poids de surfactant ; et
(c) au moins 60 % à 90 % en poids d'un solvant pharmaceutiquement acceptable ;
dans laquelle le rapport pondéral du surfactant sur le composé (1) ou un sel pharmaceutiquement acceptable de celui-ci est supérieur ou égal à 4,3 ;
et dans laquelle la composition est sensiblement dépourvue de lipide,
et dans laquelle la composition forme une dispersion transparente présentant une taille de particule moyenne inférieure à 1 µm après dilution dans un liquide gastrique simulé.

10. Composition pharmaceutique selon la revendication 9, dans laquelle le surfactant présente une balance hydrophile/lipophile supérieure à 10.

11. Composition pharmaceutique selon la revendication 9, dans laquelle le surfactant est la vitamine E-TPGS, une huile de ricin polyéthoxylée, une huile de ricin polyoxylée hydrogénée, un ester gras de polyoxyéthylène sorbitane, un caprylocaproyl-macrogol-glycéride ou un mélange de ceux-ci.

12. Composition pharmaceutique selon la revendication 9, dans laquelle le solvant pharmaceutiquement acceptable est le propylène glycol, le polypropylène glycol, le polyéthylène glycol, le glycérol, l'éthanol, la triacétine, l'isosorbide de diméthyle, le glycofurol, le carbonate de propylène, l'eau, le diméthylacétamide ou des mélanges de ceux-ci.

13. Composition pharmaceutique selon la revendication 9, dans laquelle le solvant est un mélange d'eau, de polyéthylène glycol présentant une masse moléculaire moyenne supérieure à 300 mais inférieure à 600 et de propylène glycol.

14. Composition pharmaceutique selon la revendication 9, dans laquelle la composition pharmaceutique ne contient aucun lipide.

15. Composition pharmaceutique selon la revendication 9, dans laquelle la composition pharmaceutique est sensiblement dépourvue de propylène glycol.

16. Composition pharmaceutique selon la revendication 9, dans laquelle la composition pharmaceutique est sensiblement dépourvue d'amine.
